# EUROPÄISCHE PATENTSCHRIFT

(11) **EP 1 315 515 B1**
(45) Veröffentlichungstag und Bekanntmachung des Hinweises auf die Patenterteilung: **21.11.2007**
(21) Anmeldenummer: 01982244.4
(22) Anmeldetag: 04.09.2001
(51) Int. Cl.: A61K 38/18, A61P 25/18

(54) **VERWENDUNG VON ERYTHROPOIETIN SOWIE DESSEN DERIVATE ZUR BEHANDLUNG VON SCHIZOPHRENIE UND VERWANDTEN PSYCHOSEN**
METHOD FOR TREATING SCHIZOPHRENIA AND RELATED PSYCHOSES, AND THE USE OF ERYTHROPOIETIN OR ERYTHROPOIETIN DERIVATIVES FOR TREATING SCHIZOPHRENIC DISORDERS AND RELATED PSYCHOSES
PROCEDE DE TRAITEMENT DE LA SCHIZOPHRENIE ET DES PSYCHOSES APPARENTEES, ET UTILISATION DE L'ERYTHROPOIETINE OU DES DERIVES DE L'ERYTHROPOIETINE POUR LE TRAITEMENT DES SCHIZOPHRENIES ET PSYCHOSES APPARENTEES

(30) Priorität: 04.09.2000 DE 10043457
(43) Veröffentlichungstag der Anmeldung: 04.06.2003
(73) Patentinhaber: Ehrenreich, Hannelore, 37085 Göttingen (DE)
(72) Erfinder: Ehrenreich, Hannelore, 37085 Göttingen (DE)
(74) Vertreter: Pfenning, Meinig & Partner GbR
(86) Internationale Anmeldenummer: PCT/EP2001/010198
(87) Internationale Veröffentlichungsnummer: WO 2002/020031

(56) Entgegenhaltungen:
- WO-A-00/61164
- DE-A- 19 857 609
- PATENT ABSTRACTS OF JAPAN vol. 017, no. 426 (C-1094), 9. August 1993 (1993-08-09) & JP 05 092928 A (SNOW BRAND MILK PROD CO LTD), 16. April 1993 (1993-04-16)

## Beschreibung

Die vorliegende Erfindung bezieht sich auf ein Mittel zur Behandlung von Schizophrenie unter Verwendung von Erythropoietin.

Ätiologie und Pathogenese der Schizophrenie im Sinne der obigen Definition sind bisher nicht bekannt. Zwar besteht Einigkeit über eine wichtige Rolle genetischer Einflüsse, jedoch kennt man eine Reihe von vermutlich relevanten Co-Faktoren, z.B. Neurotrauma, Drogenkonsum etc., welche den Ausbruch der Erkrankung mit zu bestimmen scheinen. Unbekannt sind insbesondere auch die molekularen und zellulären Mechanismen, welche pathogenetisch bei dieser Erkrankung eine Rolle spielen. Es gibt daher bisher auch keine wirklich guten Tiermodelle für Schizophrenie. Die vorhandenen Tiermodelle decken lediglich Teilaspekte der Erkrankung ab.

Aufgabe der vorliegenden Erfindung ist es daher, ein Mittel zur Behandlung von Schizophrenie zur Verfügung zu stellen.

Diese Aufgabe wird durch die Verwendung zur Herstellung eines Arzneimittels nach Patentanspruch 1 gelöst. Vorteilhafte Weiterbildungen der erfindungsgemäßen Verwendung werden in den abhängigen Ansprüchen gegeben.

Dabei wird unter Schizophrenie die eigentliche Schizophrenie und verwandte Psychosen verstanden. Unter Behandlung ist nicht nur die Behandlung bei bereits aufgetretenem Krankheitsbild zu betrachten, sondern auch der präventive Einsatz bei besonders gefährdeten Personen, z.B. bei Personen mit hoher erblicher Belastung, mit Neurotrauma, Psychotraume und dergleichen.

Die vorgeschlagene medikamentöse Neuroprotektion bei Schizophrenie ist dabei ein generell völlig neuer therapeutischer und prophylaktischer Ansatzpunkt. Dieser berücksichtigt die von Epidemiologen und Klinikern immer wieder bestätigte Beobachtung, daß es bereits im Verlauf der ersten Episode der Erkrankung zu einer dramatischen Beeinträchtigung der kognitiven/mentalen Leistungsfähigkeit kommt, welche zumindest partiell irreversibel ist und sich in der Regel in den weiteren Episoden eher auf gleichbleibendem oder wenig progredientem Niveau einpendelt. Hier greift nun der konsequente Einsatz eines neuroprotektiven Therapieansatzes, auch als "Add-on Therapie" in Verbindung mit einem symptomkoupierenden Neuroleptikum, besonders in der ersten Episode der Psychose ein. Auch ein präventiver Einsatz von Erythropoietin bei den besonders gefährdeten Personen wird gemäß dieser Erfindung vorgeschlagen.

Erythropoietin, auch abgekürzt als "EPO" bezeichnet, ist ein körpereigenes Glycoprotein mit einem Molekulargewicht von 34.000 Dalton (W. Jelkmann, "Erythropoietin: Structure, Control of Production, and Function", Physiological Reviews, 1992 Band 72, Seiten 449 bis 489). Es ist ein wesentlicher Wachstumsfaktor für die Produktion von Erythrozyten und wurde bereits 1977 erstmals isoliert.

Erythropoietin ist seit vielen Jahren in häufigem klinischem Gebrauch bei Patienten mit renaler Anämie bei Nephrodialyse, zur Gewinnung größerer Mengen autologen Blutes vor geplanten Operationen und geriet auch als Dopingmittel in die Schlagzeilen der Presse.

Dabei erwies sich Erythropoietin als ausgesprochen gut verträglich. Als relevante Nebenwirkung sind insbesondere die oft therapeutisch erwünschte Stimulation der Hämatopoiese mit Polyglobulie sowie eine selten zu beobachtende arterielle Hypertonie zu nennen. Beide Auswirkungen sind hauptsächlich nach chronischer Erythropoietin-Gabe zu erwarten. Ihnen ist im Bedarfsfall relativ einfach durch medikamentöse Behandlung bzw. Aderlaß abzuhelfen. Unverträglichkeitsreaktionen bzw. anaphylaktische Reaktionen gehören bei Erythropoietin zu den Raritäten.

Erythropoietin vermag als größeres Protein mit einem Molekulargewicht von ca. 34.000 Dalton bekanntermaßen gewöhnlich die Blut-Hirn-Schranke nicht zu überwinden. Eine unmittelbar intracerebroventrikuläre Gabe des Erythropoietins, d.h. unmittelbare Infusion des Erythropoietin in das Hirngewebe scheidet jedoch beim Menschen gewöhnlich aus wegen Risiken, die mit der Anlage und dem Unterhalt einer temporären Ventrikeldrainage verbunden sind, wie Infektionen oder Blutungen.

Vorteilhafter Ansatzpunkt für das erfindungsgemäße Verfahren und die erfindungsgemäße Verwendung von Erythropoietin ist es, daß bei Schizophrenie in der akuten Phase der Psychose zumeist eine Störung der Blut-Hirn-Schranke auftritt, die einen raschen Übertritt von im Blut zirkulierendem Erythropoietin ins Hirn erlaubt.

Hier setzt nun die vorliegende Erfindung sich vorteilhaft fort, indem erkannt wurde, daß im Falle einer akuten Phase einer Psychose die Blut-Hirn-Schranke für Erythropoietin durchlässig wird. Daher ist eine systemische periphere Gabe, z.B. parenteral wie vaskulär, intranasal, per Inhalation, insbesondere intravenös, subcutan und/oder intramuskulär (beispielsweise für Depotformen), des Erythropoietins dennoch erfolgreich.

Im Falle einer chronischen Behandlung bzw. Langzeittherapie findet ein langsamer Effekt der Gabe von Erythropoietin statt, sofern mit persistierend hohen Blutspiegeln (durch Gaben von bis zu 200.000 IE pro Woche) von Erythropoietin dennoch ein Übertritt des Erythropoietins über die Blut-Hirn-Schranke ins Gehirn trotz der intakten Blut-Hirn-Schranke stattfindet.

Aufgrund dieses überraschenden Übertritts von Erythropoietin in das Gehirn im Falle der akuten Phase einer Psychose bzw. durch sehr hohe Blutspiegel von Erythropoietin bei chronischer Behandlung kann Erythropoietin eingesetzt werden, um alle drei potentiell an der Pathogenese der Schizophrenie beteiligten Mechanismen, welche in einer neuronalen Dysfunktion resultieren, zu beeinflussen: Dies sind die Apoptose, die metabolische Störung der Nervenzellen sowie die synaptischen Verbindungen/axonales Sprouting.

Zusammenfassend läßt sich feststellen, daß medikamentöse Neuroprotektion bei Schizophrenie generell ein völlig neuer therapeutischer und prophylaktischer Ansatz ist.

Im folgenden werden einige Beispiele erfindungsgemäßer Verfahren und deren Ergebnisse gezeigt.

Es zeigen:

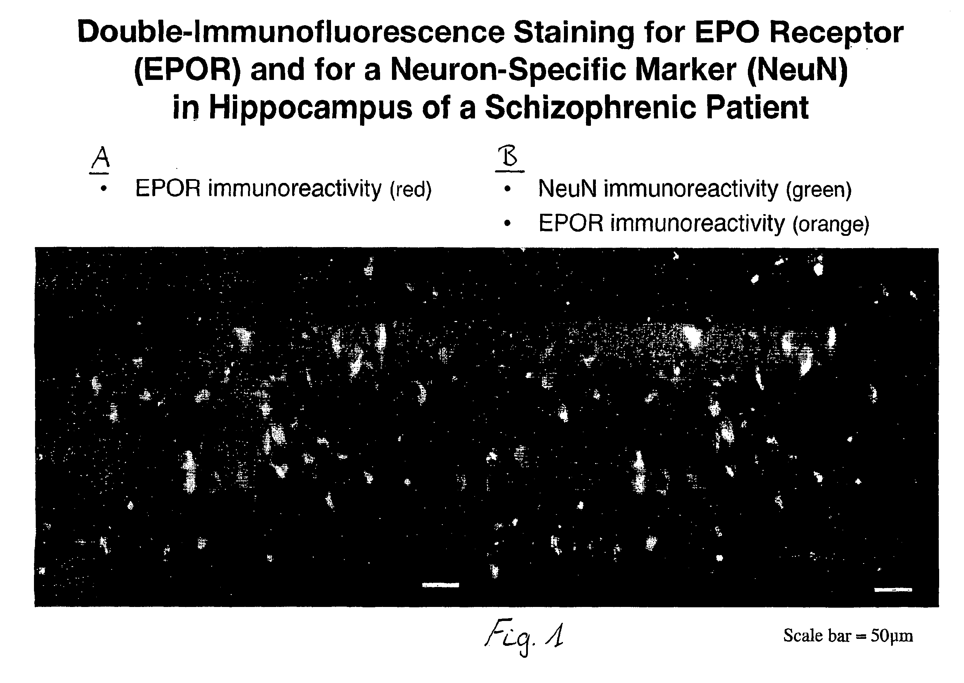

Fig. 1A und Fig. 1B immunohistochemische Untersuchungen an Gehirnschnitten Schizophrener.

In einer Versuchsserie an drei Schizophrenen im subakuten Krankheitsstadium (Erst- bzw. Zweitmanifestation) und zwei Gesunden wurde den einzelnen Patienten jeweils 40.000 i.E. Indium-111-Erythropoietin mit insgesamt 120-185 MBq einmalig intravenös appliziert. Anschliessend wurden Einzelphoton-Emissions-Computertomographien (SPECT-Aufnahmen) aufgenommen, wobei die Aufnahmen 4,18 bis 21 bzw. 42 bis 45 Stunden nach Applikation des radioaktiv markierten Erythropoietins erfolgten.

Die nachfolgende Tabelle 1 zeigt die Quotienten "mittlerer Impulsinhalt Hirn/mittlerer Impulsinhalt Knochenmark der Schädelkalotte".

| | 4h | 18-21h | 42-45h |
|---|---|---|---|
| Proband 1 | 0,69 | 0,49 | 0,46 |
| Proband 2 | 0,61 | 0,47 | 0,43 |
| Patient 1 | 0,75 | 0,59 | 0,58 |
| Patient 2 | 0,80 | 0,56 | 0,50 |
| Patient 3 | 0,76 | 0,64 | 0,49 |

Es zeigt sich an den drei Patienten 1 bis 3 mit Schizophrenie eine deutliche intracerebrale Anreicherung des radioaktiv markierten Erythropoietins zu allen drei Zeitpunkten, wobei diese Anreicherung bei den Patienten 1 bis 3 global höher ist als bei den gesunden Probanden 1 und 2. Diese Mehranreicherung des radioaktiven Erythropoietins ist aus den in der Tabelle 1 gezeigten Quotienten unmittelbar zu erkennen.

Damit wurde erstmals bewiesen, dass Erythropoietin bei Schizophrenen in der (sub)akuten Phase der Psychose die Blut-Hirn-Schranke stärker als bei Gesunden überschreitet. Darüber hinaus wurde erstmals nachgewiesen, dass Erythropoietin selbst bei Gesunden mit intakter Blut-Hirn-Schranke diese bei entsprechend hoher Dosierung zu überschreiten vermag. Somit kann eine chronische Behandlung Schizophrener auch über die akute Krankheitsphase hinweg, d.h. auch bei wieder intakter Blut-Hirn-Schranke, als neuroprotektive Add-on-Therapie genügend hohe intracerebrale Spiegel bewirken.

Erythropoietin ist dabei in der Lage, alle drei potentiell an der Pathogenese der Schizophrenie beteiligten Mechanismen, welche in einer neuronalen Dysfunktion resultieren, zu beeinflussen:
a) Apoptose;
b) Metabolische Störung der Nervenzellen;
c) Synaptische Verbindungen / axonales Sprouting.

Voraussetzung für die hier angestrebte neuroprotektive Wirkung von Erythropoietin im Gehirn Schizophrener ist dessen Bindung an spezifische Erythropoietin-Rezeptoren an Nervenzellen. Diese wurden bei den für die vorliegende Erfindung durchgeführten immunhistochemischen Studien erstmals nachgewiesen.

Fig. 1 zeigt einen histologischen Schnitt aus dem Hippocampus eines Schizophrenen (post mortem Gehirn), in welchem mittels Immunhistochemie Erythropoietinrezeptoren (EPOR: rote Färbung, linke Abbildung, Fig. 1A) und mittels Doppelfluoreszenzmethode die Lokalisation derselben an Nervenzellen nachgewiesen werden konnten (EPOR: orange Färbung, NeuN=Nervenzellmarker: grüne Färbung, rechte Abbildung, Fig. 1B). Es ist unmittelbar zu erkennen, dass die grün fluoreszierenden Nervenzellen auch mit rot fluoreszierenden EPOR-Antikörpern markiert sind, so daß sich eine orange Färbung in Fig. 1B ergibt. Mit dieser Studie wurde damit erstmals nachgewiesen, dass Nervenzellen im Gehirn Schizophrener Immunoreaktivität für Erythropoietinrezeptoren aufweisen.

Für die Figruen 1A und 1B wurden die Schnitte in Hemo-De deparaffiniert (Fisher Scientific, Schwerte, Germany), drei Waschschritte á 5 min durchgeführt, in einer absteigenden Alkoholreihe rehydriert, mit destilliertem Wasser gewaschen, in Citratpuffer gekocht, in Tris gepufferter Kochsalzlösung (TBS) gewaschen, mit 10 %igem Blockierungsserum in 0,05 % Tween-20/TBS bei Raumtemperatur inkubiert und anschließend mit einem polyklonalen Hasen-anti-humanen EPOR Antikörper (1:200, C-20, Santa Cruz Biotechnology, Heidelberg, Germany) in 2 %igem Ziegenserum/PBS bei 4 °C über Nacht inkubiert. Nach dem Waschen in 0,05 % Tween-20/TBS, wurden die Schnitte mit Texas-Red-markierten Ziege-anti-Hase Antikörpern (1:100, Vector Laboratories Inc., Burlingame, CA, USA) in einer Feuchtkammer inkubiert (30 min). Nach dem Waschen mit 0,05 % Tween-20/TBS wurden die Schnitte mit einem monoklonalen Maus-anti-neuronalen-Kern (NeuN) Antikörper (1:500, Chemicon Int. Inc., Temecula, CA, USA) in 2 %igem Pferdeserum/PBS bei +4 °C (24 h) inkubiert, in 0,05 %Tween-20/TBS gewaschen und in einer Feuchtkammer mit Fluorescein (FITC) markierten Pferde-anti-Maus Antikörpern (1:100, Vector) für 30 min inkubiert. Die Schnitte wurden anschließend in 0,05 % Tween-20/TBS und TBS gewaschen und schließlich in Vectashield (Vector) Fluoreszenzmedium eingebettet.

## Patentansprüche

1. Verwendung von Erythropoietin zur Herstellung eines peripher zu applizierenden Arzneimittels zur Behandlung und/oder Prophylaxe von Schizophrenie in einer Dosis von 5000 I.E. bis 200000 I.E. pro Gabe und/oder pro Tag und/oder pro Woche, mit der Maßgabe, dass eine Dosis von 50000 I.E. oder mehr ausgenommen ist.

2. Verwendung nach dem vorhergehenden Anspruch zur Herstellung eines vaskulär zu applizierenden Arzneimittels.

3. Verwendung nach dem vorhergehenden Anspruch zur Herstellung eines intravenös zu applizierenden Arzneimittels.

4. Verwendung nach einem der vorhergehenden Ansprüche in einer Dosis von 35.000 IE pro Gabe und/oder Tag.

5. Verwendung nach einem der vorhergehenden Ansprüche, **dadurch gekennzeichnet, daß** als Erythropoietin ein natives oder rekombinantes menschliches oder tierisches Erythropoietin oder ein Derivat hiervon verwendet wird.

6. Verwendung nach einem der vorhergehenden Ansprüche, **dadurch gekennzeichnet, dass** das Säugetier ein Mensch ist.

## Claims

1. Use of erythropoietin for producing a medicine to be applied peripheral to the treatment and/or prophylaxis of schizophrenia at a dose of 5000 I.E. to 200000 I.E. per administration and/or per day and/or per week, with the provision that a dose of 5000 I.E. or more is excepted.

2. Use according to the preceding Claim for producing a medicine to be administered vascularly.

3. Use according to the preceding Claim for producing a medicine to be administered intravenously.

4. Use according to one of the preceding Claims in the form of a dose of 35000 I.E. per administration and/or day.

5. Use according to one of the preceding Claims, **characterised in that** a native or recombined human or animal erythropoietin or a derivative of the same is used as erythropoietin.

6. Use according to one of the preceding Claims, **characterised in that** the mammal is a human.

## Revendications

1. Utilisation d'érythropoïétine pour la fabrication d'un médicament à administrer par voie périphérique, destiné au traitement et/ou à la prophylaxie de la schizophrénie à une dose de 5 000 UI à 200 000 UI par prise et/ou par jour et/ou par semaine, étant entendu qu'une dose de 50 000 UI ou plus est exclue.

2. Utilisation selon la revendication précédente, pour la fabrication d'un médicament à administrer par voie vasculaire.

3. Utilisation selon la revendication précédente, pour la fabrication d'un médicament à administrer par voie intraveineuse.

4. Utilisation selon l'une quelconque des revendications précédentes, à une dose de 35 000 UI par prise et/ou par jour.

5. Utilisation selon l'une quelconque des revendications précédentes, **caractérisée en ce qu'**on utilise en tant qu'érythropoïétine une érythropoïétine humaine ou animale, native ou recombinante, ou un dérivé de celle-ci.

6. Utilisation selon l'une quelconque des revendications précédentes, **caractérisée en ce que** le mammifère est un sujet humain.
